# EUROPEAN PATENT SPECIFICATION

(11) **EP 4 512 317 B1**
(45) Date of publication and mention of the grant of the patent: **13.05.2026**
(21) Application number: 24181720.4
(22) Date of filing: 12.06.2024
(51) Int. Cl.: A61B 5/021, A61B 5/024, A61B 5/00

(54) **A WEARABLE DEVICE FOR MEASURING A BLOOD PRESSURE OF A WEARER**
TRAGBARE VORRICHTUNG ZUR MESSUNG DES BLUTDRUCKS EINES TRÄGERS
DISPOSITIF PORTABLE POUR MESURER LA PRESSION SANGUINE D'UN UTILISATEUR

(30) Priority: 23.08.2023 NL 2035661
(43) Date of publication of application: 26.02.2025
(73) Proprietor: Wear It System Holding S.A., 1228 Plan- les- Ouates (CH)
(72) Inventor: HANSE, Iddo Johanan Gideon, 1228 Plan- les- Ouates (CH); EBRAHIMKHEIL, Kambiz, 1228 Plan- les- Ouates (CH)
(74) Representative: van Breda, Jacobus

(56) References cited:
- US-A1- 2023 082 362
- MOAJJEM HOSSAIN CHOWDHURY ET AL: "Estimating Blood Pressure from Photoplethysmogram Signal and Demographic Features using Machine Learning Techniques", ARXIV.ORG, CORNELL UNIVERSITY LIBRARY, 201 OLIN LIBRARY CORNELL UNIVERSITY ITHACA, NY 14853, 7 May 2020 (2020-05-07), XP081670194
- AKUTHOTA UDAY CHANDRA ET AL: "Blood Pressure Prediction Based on Single Photoplethysmography", 2022 IEEE INTERNATIONAL SYMPOSIUM ON SMART ELECTRONIC SYSTEMS (ISES), IEEE, 18 December 2022 (2022-12-18), pages 337 - 342, XP034286090, DOI: 10.1109/ISES54909.2022.00075
- EL-HAJJ C ET AL: "A review of machine learning techniques in photoplethysmography for the non-invasive cuff-less measurement of blood pressure", BIOMEDICAL SIGNAL PROCESSING AND CONTROL, ELSEVIER, AMSTERDAM, NL, vol. 58, 11 February 2020 (2020-02-11), XP086065092, ISSN: 1746-8094, [retrieved on 20200211], DOI: 10.1016/J.BSPC.2020.101870

## Description

A wearable device for measuring a blood pressure of a wearer The invention relates to a wearable device for non-invasively measuring blood pressure using multi-wavelength photoplethysmography (PPG) signals obtained by said wearable device from peripheral blood vessels.

### General Description

Photoplethysmography (PPG) is a non-invasive optical technique that measures blood volume changes in the microvascular bed of tissue. It works by shining a light source, typically an LED, onto the skin and detecting the amount of light that is transmitted or reflected back to a photodetector. This optical signal can be used to derive information about blood flow, heart rate, and other physiological parameters.

Blood pressure estimation based on photoplethysmography (PPG) signals has received increasing attention in recent years due to the non-invasive and continuous nature of PPG measurements. There are a variety of algorithms that have been proposed for estimating blood pressure from PPG signals, including methods that utilize waveform-based feature extraction, frequency features of the input signal, and combinations of both. Some of these methods also incorporate other physiological signals, such as electrocardiogram (ECG) and PPG sensors from multiple locations on the body, to obtain an indication of Pulse-Transit Time (PPT), a feature highly correlated with blood pressure. Although accurate, having sensors placed on multiple locations of the body imposes new challenges, such as placement determination and an increased chance of noisy signals.

Moajjem Hossain Chowdhurry et al. in their article in Sensors 2020, titled "Estimating Blood Pressure from Photoplethysmogram Signal and Demographic Features using Machine Learning Techniques", proposed a method for estimating blood pressure (BP) based on PPG signals wherein time, frequency and time-frequency domain features are extracted from the PPG signals and fed in to ML algorithms to estimate systolic BP and diastolic BP. US20230082362 and Akuthota Chandra et al. in their publication in IEEE 2022 proposed measuring BP from single PPG.

El-Hajj et al. in their publication in biomedical signal processing and control 2020, proposed using machine learning and PPG signal processing to estimate blood pressure.

The existing single measurement site PPG-based blood pressure algorithms are based on single-wavelength signal, and use time- and frequency domain features in order to estimate blood pressure. These features are often used as input in machine learning methods to train a model that maps the combination of features to the associated systolic and diastolic blood pressure values. A problem with only using time- and frequency domain features based on the direct ppg signal is a high overlap and correlation of features, making the algorithms prone to overfitting on certain characteristics in the PPG signal.

It is an object of the current invention to correct the shortcomings of the prior art and to provide a solution for measuring a blood pressure of a wearer using single-site measurements while providing measurements accuracy better than that of multiple-site measurements.

This and other objects which will become apparent from the following disclosure, are provided with a wearable device having the features of one or more of the appended claims.

In a first aspect of the invention, the wearable device is configured for processing a photoplethysmography signal and for emitting light at a plurality of light-wavelengths for measuring a blood pressure of a wearer of said device, wherein the photoplethysmography signal comprises a plurality of pulses and wherein a pulse is the photoplethysmography signal between two consecutive valleys, wherein, for each light-wavelength, the wearable device is configured for:
- creating a photoplethysmography segment by acquiring the photoplethysmography signal within a predefined time window;
- collecting at least one biologically vital sign, such as a heart rate of the wearer, a respiration rate of the wearer, and an oxygen saturation rate of the wearer;
- collecting at least one waveform-based parameter proportional to a peak amplitude of said photoplethysmography segment;
- collecting at least one time-based parameter proportional to a peak time of said photoplethysmography signal or proportional to a peak width of said photoplethysmography segment;
- creating a cardiovascular profile vector of the wearer;
- calculating a mean squared error between the collected profile vector and a profile vector belonging to previously predicted cluster of profile vectors, not necessarily profile vectors of the current wearer; and
- estimating a blood pressure of the wearer using the photoplethysmography segments comprised in the cardiovascular profile of the wearer when said mean squared error is smaller than a first threshold value.

Advantageously, the wearable device is configured for filtering the photoplethysmography segment, preferably by using a bandpass filter, and using said filtered photoplethysmography segments in the remaining steps of the method.

More advantageously, the wearable device is configured for creating a cardiovascular profile vector of the wearer comprises concatenating the collected at least one waveform-based parameter, the collected at least one time-based parameter, the collected at least one biologically vital sign and corresponding non-eliminated photoplethysmography segments into a single profile vector.

Multi-wavelength PPG sensors have the advantage of being able to measure changes in different wavelengths of light, which can provide more information about the hemodynamic changes occurring in tissue. For example, different wavelengths of light can penetrate to different depths in tissue, allowing for measurements of both arterial and venous blood flow. Additionally, different wavelengths of light are absorbed differently by different chromophores, such as oxygenated and deoxygenated hemoglobin, which can provide insights into tissue oxygenation and metabolism. By combining information from multiple wavelengths, multi-wavelength PPG sensors can provide a more comprehensive picture of cardiovascular function and tissue perfusion, which are used to calculate informative features from the PPG signal, to use as input for the blood pressure estimation algorithm. Existing works do not address the inter-wavelength characteristics of PPG signals and their predictive capabilities to estimate blood pressure.

Furthermore, a well-known problem in PPG-based solutions is the capricious nature of the signal, meaning that the waveform of the PPG signal can display sudden changes in characteristics in a relatively short amount of time. This poses a challenge for beat-to-beat prediction in real-life settings. To overcome this problem, the current invention is based on set timeframes of PPG signals, in which a multi-wavelength signal quality indication method isolates only the useful and high-quality signals. These signals are then used to calculate both waveform-based parameters, time-based parameters, and a combination of the two. Advantageously, the wearable device is optionally configured for normalizing the collected at least one time-based parameter by multiplying said at least one time-based parameter by a factor inversely proportional to a peak width of the photoplethysmography segment. This ensures consistency across different sensors or patients, helping to maintain accuracy when the data is analyzed and compared.

Suitably, the wearable device is configured to emit light comprising light-wavelengths between 400 nm and 1000 nm.

More suitably, the wearable device is configured to emit light comprising:
- a light-wavelength of 525 nm;
- a light-wavelength of 660 nm; and
- a light-wavelength of 880 nm.

In real-world scenarios, not all collected PPG signals are of high quality. Various factors, such as patient movement or sensor placement, may degrade the signal quality, leading to erroneous feature extraction and thus inaccurate blood pressure predictions. To mitigate this, our system employs a Signal Quality Indicator (SQI). Advantageously, for each photoplethysmography segment, the wearable device is preferably configured for:
- calculating at least one of the following values for each pulse of said photoplethysmography segment:
   ∘ a pulse wave amplitude left by calculating a difference between vertical amplitudes of a first peak and a first valley of the pulse;
   ∘ a pulse wave amplitude right by calculating a difference between vertical amplitudes of the first peak and a second valley of the pulse;
   ∘ a pulse wave duration by calculating a difference between a time of the second valley and a time of the first valley of the pulse;
   ∘ a rise time by calculating a difference between a time of the first peak and a time of the first valley of the pulse;
   ∘ a systolic-to-diastolic duration ratio by calculating the ratio between a difference between the rise time and a difference between the time of the second valley and the time of the first peak of the pulse;
- eliminating a photoplethysmography pulse when:
   ∘ the rise time of said pulse is outside a predefined first range, optionally [0.01 - 10] second or [0.08 - 0.49] seconds; or
   ∘ the pulse wave duration of said pulse is outside a predefined second range, optionally [0.01 - 10] second or [0.3 - 2] seconds; or
   ∘ a ratio or an inverse ratio between the pulse wave amplitude right and the pulse wave amplitude left is smaller than a second threshold value e.g. 50%; or
   ∘ the systolic-to-diastolic duration ration is larger than a third threshold optionally equal to 5 or 3 or 1.1;
- calculating a signal quality index of the photoplethysmography segment by calculating a ratio of non-eliminated photoplethysmography pulses over a total number of photoplethysmography pulses comprised in said photoplethysmography segment.

Furthermore, the wearable device is preferably configured for eliminating the photoplethysmography segment when signal quality index of said photoplethysmography signal is lower than a fourth threshold value. The wearable device is preferably configured for setting said fourth threshold value at 20%. Only PPG segments having an SQI higher than the fourth threshold value (e.g. higher than 20%) are retained for further processing. This quality control step is crucial as it ensures the reliability and accuracy of the subsequent feature extraction and blood pressure prediction stages. By rejecting poor quality signals at an early stage, the system significantly reduces the chances of model contamination and error propagation.

To further adapt the PPG measurements, and subsequently the blood pressure predictions, to the unique physiology of the wearer, the wearable device is preferably configured for:
- collecting the wearer demographic information such as age, sex, body weight and height; and
- adding said demographic information into the cardiovascular profile of the wearer.

Advantageously, the wearable device is configured for grouping profile vectors into a plurality of clusters based on similarity in at least one of the vital signs and on similarity in the wearer demographic information by a using a silhouette coefficient to determine an optimal number of clusters for establish a K-Means clustering on said profile vectors.

Additionally, The wearable device is preferably configured for training a Random Forest model on each cluster by combining a plurality of decision trees and training each decision tree on a separate subset data of said cluster. In fact, the wearable device is preferably configured for employing a variety of machine learning techniques for model training and calibration. After preprocessing, normalization, and scalarization of the input data, a classification model identifies the appropriate user subgroup based on demographic features and features extracted from the initialization measurements. The predictive model, trained using a piecewise ensemble method, utilizes the cross-validated mean-squared error, or cross-entropy, or a combination thereof as a loss function. This involves splitting the training data into multiple subsets, training the ensemble model on a subset, and testing it on the remaining data. This process is iteratively repeated with different data subsets to robustly evaluate model performance.

The cross-validated mean squared error, for example, is computed by averaging the squared differences between predicted and actual blood pressures across all subsets. This error squaring emphasizes larger errors and guarantees positive values. The cross-validated mean squared error offers a comprehensive performance metric for the model. The trained model is then used to map input features into numerical values that yield the predicted systolic and diastolic blood pressure readings.

Suitably, the wearable device is preferably configured for:
- assigning a new wearer to one of the plurality of clusters based on the demographic information of the new wearer and based on at least one of the vital signs of said new wearer; and
- using the Random Forest model corresponding to said cluster as a starting point, and fine-tuning said starting point to create a cardiovascular profile for the new wearer using a regularization framework such as a Lasso regression, a Ridge regression or an Elastic Net.

More suitably, the wearable device is preferably configured for updating the cardiovascular profile of the wearer by performing the steps of the preceding claims on new sets of acquired photoplethysmography segments. This is for creating a patient-specific model that represents a fusion of generalized cluster-based learning and personalized fine-tuning.

In a second aspect of the invention, the wearable device configured to emit light onto a skin of a wearer and to receive light emitted from the skin of the wearer wherein the device comprises a computer system capable of performing the steps according to any one of the aforementioned aspects.

### Detailed description

The invention will hereinafter be further elucidated with reference to the drawing of an exemplary embodiment of the operation of a wearable device according to the invention that is not limiting as to the appended claims.

In the drawing,
- figure 1 shows a first diagram of the operation of the wearable device of the invention;
- figure 2 shows a second diagram of the operation of the wearable device of the invention;
- figure 3 shows a third diagram of the operation of the wearable device of the invention;
- figure 4 shows graphs related to the operation of the wearable device of the invention.

### Data Acquisition and Preprocessing:

The wearable device of the invention takes in various forms of data such as demographic details (like age, sex, weight, height and skin color), vital signs (including heart rate, respiratory rate and SpO2) and PPG signals. These PPG signals are captured at a plurality of light-wavelengths, in particular at three distinct wavelengths, namely red (660 nm), green (525 nm), and infrared (880 nm). The selection of these specific wavelengths is strategic as they each penetrate the skin at different depths, allowing for the capture of arterial pulsations on multiple levels. This multi-level capture improves the reliability of the signal and reduces the impact of motion artifacts, an important feature for practical usability of the system.

Following the collection of raw PPG signals, the data undergoes a preprocessing phase. During this phase, a bandpass filter such as a Chebyshev filter with a predefined general range of e.g. [0.3 - 5] Hz is applied to remove the high-frequency noise and baseline wander from the signals. The exact frequencies that are filtered out are determined by the corresponding vital signs to the PPG segment. For example, the respiration rate generally causes a baseline wander between 0.1-0.6 Hz, for which the corresponding frequency is filtered out of the ppg signal. The signals are normalized to a uniform scale. This ensures consistency across different sensors or patients, helping to maintain accuracy when the data is analyzed and compared.

### Signal Quality Assessment:

In real-world scenarios, not all collected PPG signals are of high quality. Various factors, such as patient movement or sensor placement, may degrade the signal quality, leading to erroneous feature extraction and thus inaccurate blood pressure predictions. To mitigate this, the method of the current invention employs a Signal Quality Indicator (SQI).

Only PPG segments having a signal quality index higher than the 4^{th} threshold value (e.g. 20%) are retained for further processing. This quality control step is crucial as it ensures the reliability and accuracy of the subsequent feature extraction and blood pressure prediction stages. By rejecting poor quality signals at an early stage, the system significantly reduces the chances of model contamination and error propagation.

### PPG parameters Extraction:

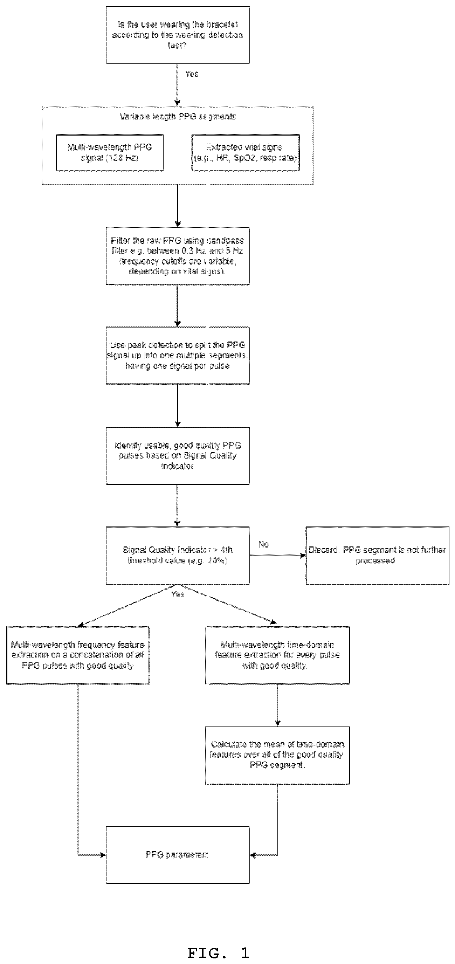

In the post-preprocessing phase, the wearable device of the current invention is optionally configured for extracting time-based parameters and waveform-based parameters from each PPG wavelength signal and a combination of PPG wavelength signals. This includes information like pulse amplitude, peak-to-peak intervals, signal energy, spectral entropy, and frequency peaks from spectral analysis. These features, combined from all three wavelengths, are then concatenated into PPG parameters vectors. This set serves as a robust representation of the wearer's cardiovascular profile, capturing both the temporal and spectral dynamics of the cardiovascular system. An extensive list of waveform-parameter and time-based parameters is given in appendix 1. Figure 1 illustrates the step of data acquisition and preprocessing, the step of calculating the SQI, and the step extracting PPG parameters.

### Cluster Identification:

Once a plurality of wearers' cardiovascular profiles are created, a K-Means clustering algorithm is employed to obtain clusters of ppg segments that contain datapoints that are similar to each other in terms of vital signs and patient demographics. Since the relationship between the characteristics of the PPG signal (features) and the blood pressure is different under different conditions, these clusters are differentiated in order to train the parameters for the prediction model for each cluster.

K-Means is a versatile algorithm, capable of effectively partitioning data into distinct groups based on their similarities. To determine the optimal number of clusters (K), the Silhouette Coefficient is used. This metric provides a measure of how similar an object is to its own cluster compared to other clusters. In this way, the computer-implemented method is able to segregate the wearers population into distinct cardiovascular profiles, improving the homogeneity of the groupings, which is a key factor in the accuracy of the subsequent predictive models.

### Cluster-Based Model Training:

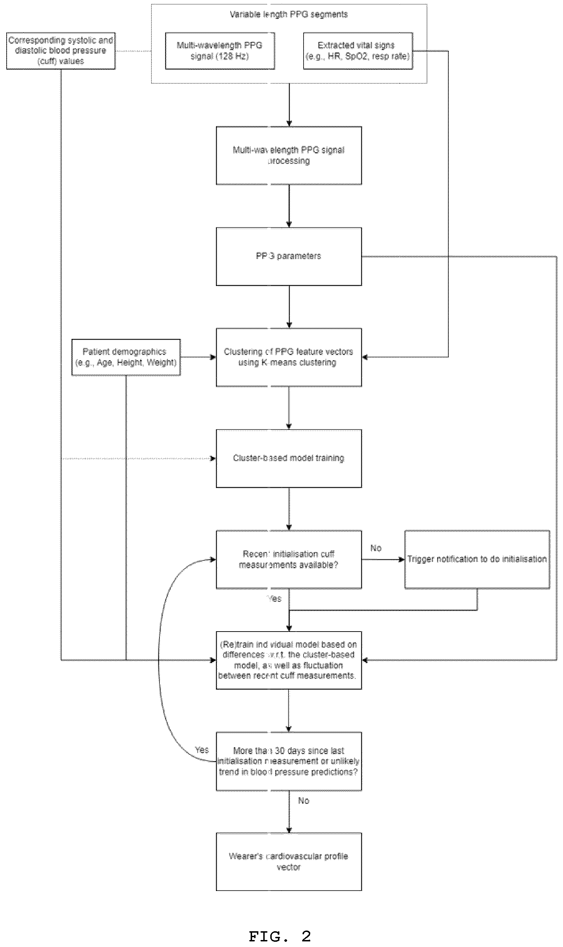

For each identified cluster, the wearable device is configured for training a Random Forest model. This model type is specifically chosen for its ability to handle high-dimensional data and its inherent resistance to overfitting through ensemble learning. It essentially combines multiple decision trees, each trained on a different subset of the data, thereby improving model robustness and predictive accuracy. The most important features differ per cluster, which are automatically extracted by the random forest ensemble technique. This ensures a limited selection of the extensive feature list per cluster model. During training, the wearable device is configured for optimizing model hyperparameters through a technique called grid search, combined with a 5-fold cross-validation setup. This rigorous approach helps balance model complexity with prediction accuracy, a crucial aspect in building effective predictive models. The cluster identification process and the cluster-based training are illustrated in figure 2.

### Personalized Model Training:

Whenever new wearers is introduced to the database, they are assigned to one of the pre-identified clusters. This assignment is based on their demographic, vital signs, and PPG features. The system then uses the corresponding cluster's Random Forest model as a foundational structure, and fine-tunes it to the wearer's unique physiology. Here, the computer-implemented method comprises the step of using a regularization framework such as a Lasso model, known for its regularization properties that encourage a sparse solution, thus effectively preventing overfitting. In this manner, the method of the current invention comprises the step of creating a wearer-specific model that represents a fusion of generalized cluster-based learning and personalized fine-tuning.

### Personalized Model Updating:

The wearable device is configured to continuously learn and adapt. As new calibration data is received from the wearer, which includes concurrent PPG signals and blood pressure measurements, the computer-implemented method comprises the step of performing incremental updates on the patient-specific regularization framework parameters. This enables the model to adapt to the wearer's unique and evolving cardiovascular dynamics, thereby maintaining accuracy over time.

### Prediction:

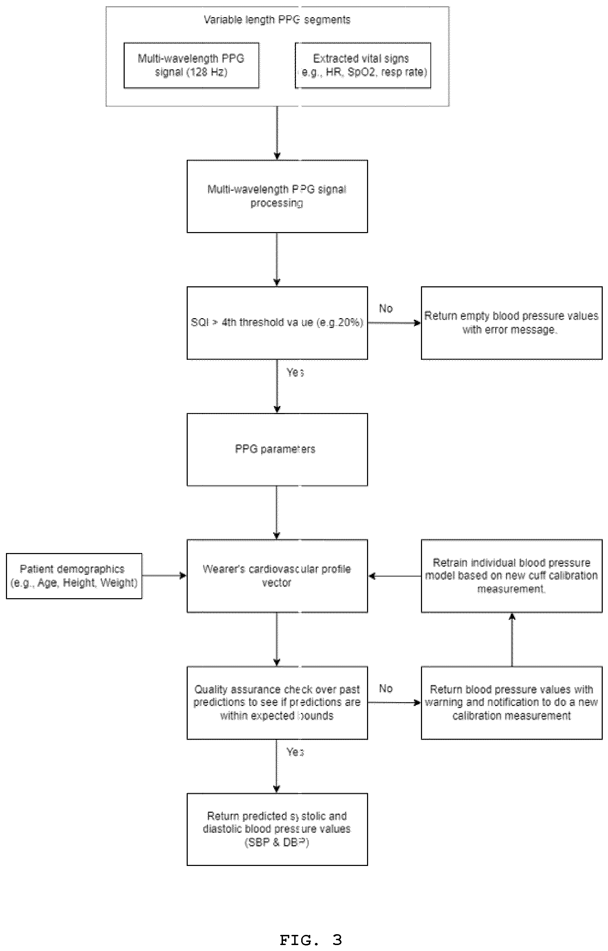

The fine-tuned wearer-specific regularization framework is used by the system to predict blood pressure. In cases where calibration data is scarce or insufficient for comprehensive fine-tuning, the system reverts to the cluster-based Random Forest model, thereby ensuring reliable and consistent predictions at all times. The personalized model training, the personalized model updating and the prediction process are illustrated in figure 3.

Different vitals do not affect the PPG signal in itself so much, but rather the interaction between the PPG signal and the blood pressure. The majority of the information is not found in differences in PPG features, but rather in the interaction effect between vital signs and PPG features.

The clustering is therefore not just used for how the signals are filtered, but the more important part is how they are handled in the rest of the algorithm.

For example, the PPG signal and its corresponding feature values at a moment of very high heart rate for a person with low blood pressure might be very similar to the PPG feature values seen in a measurement for a person with high blood pressure that is in a lower heart rate region. As these interactions effects are explained by big differences in vital signs, it is important to classify mentioned PPG segments into different groups of activity level (respiration rate, heart rate, blood oxygen saturation).

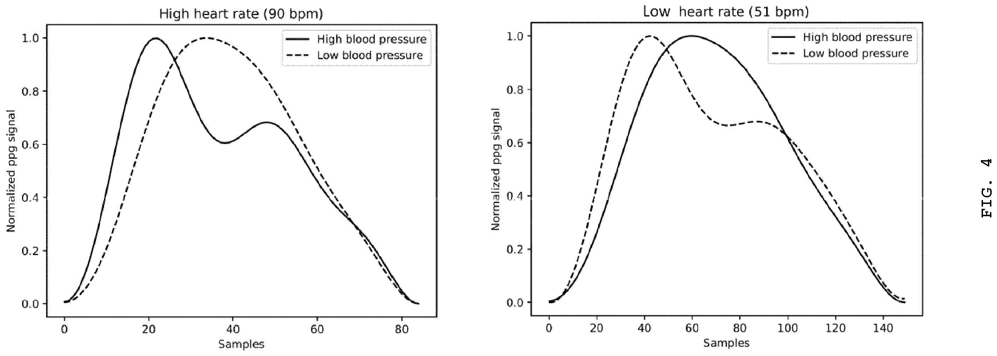

To better understand how heart rate could affect the PPG signal under multiple conditions, figure 4 illustrates four PPG curves that have been found in a patient in multiple conditions. As shown in figure 4, the PPG signal corresponding to a measurement with high BP and low HR (heartrate) looks very much like the signal corresponding to a measurement with low BP and high HR. In addition to controlling for the difference in absolute length of the signal (+- 150 samples in low HR condition vs. +- 85 samples in high HR condition) by normalizing the calculated features, there are more interaction effects at play.

The novelty of the wearable device of the current invention lies in the interaction effect between the extracted PPG parameters and blood pressure under different settings of vitality. The wearable device is optionally configured for training multiple models corresponding to distinct combinations of biologically vital signs and/or demographic ranges, thereby accounting for different physiological and lifestyle factors that might influence blood pressure.

Ranges for the biologically vital signs, such as heart rate, respiration rate, and blood saturation can be determined by applying a clustering algorithm to the data of a plurality of wearers. This ensures an adaptive segmentation of vital signs according to the underlying distribution of wearers.

The demographics data, including age, BMI (Body Mass Index), and gender, can also be divided into specific ranges. These are defined according to conventional categories.
- BMI: underweight, normal weight, overweight, obese I, obese II, obese II;
- Age: (0, 18), (19, 30), (31, 45), (46, 55), (56, 65), (66, 75), (75, 85), (85, 100);
- Gender: Male / female.

The wearable device is configured for training individual models for every unique combination of the defined ranges of biologically vital signs and/or demographics. This results in a multi-dimensional matrix of models, allowing for a high degree of specificity in capturing the complex interactions between these variables and blood pressure. The training process comprises the following steps:
a. Filtering Data: The data is filtered according to each specific combination of ranges, resulting in subsets of the data that correspond to each model.
b. Model Training: A predictive model is trained on each subset of the data, with blood pressure as the target variable.
c. Model Saving: Trained models are saved, indexed by the corresponding combination of ranges, for subsequent retrieval during prediction.

The prediction process can handle new PPG data with or without accompanying demographic information. The steps comprise:
a. Identifying Ranges: The corresponding ranges for the vital signs and available demographics are identified using the same clustering or segmentation logic applied during training.
b. Utilizing Default Demographics: If demographic data is not provided, the default values for age, BMI, and gender are calculated by taking the mean range from the training data with ppg data that has the most similar pattern to the current window. These values are used when (some of the) demographic information is not available during prediction.
c. Model Loading: The specific model corresponding to the identified combination of vital signs and demographics ranges is loaded.
d. Prediction: The loaded model is used to predict the blood pressure based on the incoming PPG data.

The wearable device of the current invention offers a versatile and highly specific approach to blood pressure prediction, accommodating complex interactions between biologically vital signs, PPG features, and demographic factors. By segmenting the data into ranges defined by clustering and training distinct models for each combination, the method provides personalized and nuanced insights into blood pressure behavior. The ability to handle missing demographic data adds to its adaptability and broad applicability in various medical and healthcare settings. In other words, the method of the current invention combines the strengths of generalized models derived from clustered population data with the advantages of personalized models incrementally fine-tuned on individual calibration data. By harnessing PPG signals of multiple wavelengths and adopting a continuous learning approach, it offers an inclusive, robust solution that is adaptable to diverse patient characteristics and continuously enhances its accuracy as it processes more data.

Although the invention has been discussed in the foregoing with reference to an exemplary embodiment of the wearable device of the invention, the invention is not restricted to this particular embodiment which can be varied in many ways without departing from the invention. The discussed exemplary embodiment shall therefore not be used to construe the appended claims strictly in accordance therewith. On the contrary the embodiment is merely intended to explain the wording of the appended claims without intent to limit the claims to this exemplary embodiment.

The scope of protection of the invention shall be construed in accordance with the appended claims only.

### Appendix 1

From the PPG segments, time-based and waveform-based parameters are extracted to be used as input for the machine learning model. To get a valid prediction, a combination of statistical features, frequency domain features, demographic features, first/second derivative features, width related PPG features and features from the PPG signal are used. All time-domain features are scaled by dividing by the length of the ppg-pulse, to eliminate the effect of heartrate on the trained models, as the heartrate is extremely correlated with any time-domain features.

List of collected and extracted features:
- Systolic peak,
- Diastolic peak,
- Height of Notch,
- Systolic peak time (spt),
- Diastolic peak time (dpt),
- Height of Notch Time (hnt),
- Time delta (dt),
- Pulse interval (pi)
- Peak-to-peak interval (ppi)
- Pulse width
- Inflection point area
- Augmentation index
- Alternative augmentation index
- Systolic peak output curve
- Diastolic peak downward curve
- Height of signal at 25% of the systolic peak downward curve (h25_sdt)
- Height of signal at 50% of the systolic peak downward curve (h50_sdt)
- Height of signal at 75 % of the systolic peak downward curve (h75_sdt)
- h25_sdt / h50_sdt
- h25_sdt - h50_sdt
- (spt)/(ppi)
- (hnt)/(ppi)
- (dpt)/(ppi)
- (dt )/(ppi)
- (Height of Notch)/(Systolic peak)
- (hnt)/(Height of Notch)
- (dpt)/(Diastolic peak)
- (Systolic peak)/(pi-spt)
- (Systolic peak)/(pi-hnt)
- Waveform width at 25% amplitude of systolic peak (Width 25%)
- Waveform width at 50% amplitude of systolic peak (Width 50%)
- Waveform width at 75% amplitude of systolic peak (Width 75%)
- Diastolic width at 25% amplitude of systolic peak (w25_sdt)
- Diastolic width at 50% amplitude of systolic peak (w50_sdt)
- Diastolic width at 75% amplitude of systolic peak (w75_sdt)
- Diastolic width at 90% amplitude of systolic peak (w90_sdt)
- Width(25%)IR / Roundness of systolic peakG
- h25_sdtR / h25_sdtIR
- h25_sdtR / h50_sdtIR
- h50_sdtG / h25_sdtIR
- Diastolic_peakR / Diastolic_peakIR
- Diastolic_peakR / Diastolic_peakG
- Diastolic_peakIR / Diastolic_peakG
- Diastolic_peakIR / Diastolic_peakR
- Roundness of systolic peak (Waveform AUC at 75% of systolic amplitude)
- (Width 25%)/spt
- (Width 25%)/(hnt)
- (Width 25%)/(dpt)
- (Width 25%)/(dt)
- (Width 25%)/(pi)
- (Width 50%)/(spt)
- (Width 50%)/(hnt)
- (Width 50%)/(dpt)
- (Width 50%)/(dt)
- (Width 50%)/(pi)
- (Width 75%)/(spt)
- (Width 75%)/(hnt)
- (Width 75%)/(dpt)
- (Width 75%)/(dt)
- (Width 75%)/(pi)
- First max of first derivative(a_fir)
- Position first max of first derivative (t_a_fir)
- First max of second derivative (a_sec)
- Position first max of second derivative(t_a _sec)
- First minimum peak first derivative of the PPG waveform after a1 (b_fir)
- Interval from foot of PPG waveform to b_fir (tb_fir)
- First minimum peak first derivative of the PPG waveform after a1 (b_sec)
- Interval from foot of PPG waveform to b_sec (tb_sec)
- (b_sec)/(a_sec)
- (b_fir)/(a _fir)
- (ta_fir)/(ppi)
- (tb_fir)/(tpp)
- (ta_sec)/(ppi)
- (tb_sec)/(ppi)
- (ta_fir-ta_sec)/(ppi)
- (tb_fir-tb_sec)/(ppi)
- (Height)/(dt)
- (Weight)/(dt)
- (BMI)/(dt)
- (Height)/(spt)
- (Weight)/(spt)
- (BMI)/(spt)
- (Height)/(hnt)
- (Weight)/(hnt)
- (BMI)/(hnt)
- (Height)/(dpt)
- (Weight)/(dpt)
- (BMI)/(dpt)
- (Height)/(pi)
- (Weight)/(pi)
- (BMI)/(pi)
- (Height)/(ppi)
- (Weight)/(ppi)
- (BMI)/(pi)
- Mean
- Median
- Standard Deviation
- Percentile
- Mean Absolute Deviation
- Inter Quartile Range
- Skewness
- Kurtosis
- Shannon Entropy
- Amplitude first peak Fast Fourier Transform PPG signal (P1)
- Amplitude second peak Fast Fourier Transform PPG signal (P2)
- Frequency first peak Fast Fourier Transform PPG signal (F1)
- Frequency second peak Fast Fourier Transform PPG signal (F2)
- Area under the curve from 0 to 2 Hz fast Fourier transform PPG signal (AUC0-2)
- Area under the curve from 2 to 5 Hz fast Fourier transform PPG signal (AUC2-5)
- (AUC0-2)/(AUC2-5)
- (P1)/(P2)
- (F1)/(F2)
- Maximum Frequency in signal spectrum
- Magnitude at maximum frequency
- Spectral Entropy

## Claims

1. A wearable device for processing a photoplethysmography signal wherein said wearable device is configured for emitting light at a plurality of light-wavelengths for measuring a blood pressure of a wearer of said device, wherein the photoplethysmography signal comprises a plurality of pulses and wherein a pulse is the photoplethysmography signal between two consecutive valleys, **characterized in that,** for at least one light-wavelength, the wearable device is configured for:
- creating a photoplethysmography segment by acquiring the photoplethysmography signal within a predefined time window;
- collecting at least one biologically vital sign, such as a heart rate of the wearer, a respiration rate of the wearer, or an oxygen saturation rate of the wearer;
- collecting at least one waveform-based parameter proportional to a peak amplitude of said photoplethysmography segment;
- collecting at least one time-based parameter proportional to a peak time of said photoplethysmography signal or proportional to a peak width of said photoplethysmography segment;
- creating a cardiovascular profile vector of the wearer;
- calculating a mean squared error between components of the collected profile vector and components of a profile vector belonging to previously predicted cluster of profile vectors; and
- estimating a blood pressure of the wearer using the photoplethysmography segments comprised in the cardiovascular profile of the wearer when said mean squared error is smaller than a first threshold value.

2. The wearable device according to claim 1, **characterized in that** the wearable device is configured for filtering the photoplethysmography segment, preferably by using a bandpass filter, and using said filtered photoplethysmography segment in the remaining steps of said method.

3. The wearable device according to claim 1 or 2, **characterized in that** the wearable device is configured for creating a cardiovascular profile vector of the wearer by concatenating photoplethysmography segments with at least one of:
- the collected at least one waveform-based parameter of the wearer;
- the collected at least one time-based parameter of the wearer; and
- the collected at least one biologically vital sign of the wearer.

4. The wearable device according to any one of the preceding claims, **characterized in that** the wearable device is configured for normalizing the collected at least one time-based parameter by multiplying said parameter by a factor inversely proportional to a peak width of the photoplethysmography segment.

5. The wearable device according to any one of the preceding claims, **characterized in that** the wearable device is configured to emit light comprising light-wavelengths between 400 nm and 1000 nm.

6. The wearable device according to any one of the preceding claims, **characterized in that** the wearable device is configured to emit light comprising:
- a light-wavelength of 525 nm;
- a light-wavelength of 660 nm; and
- a light-wavelength of 880 nm.

7. The wearable device according to any one of the preceding claims, **characterized in that,** for at least one photoplethysmography segment, the wearable device is configured for:
- calculating at least one of the following values for each pulse of said photoplethysmography segment:
∘ a pulse wave amplitude left by calculating a difference between amplitudes of a first peak and a first valley of the pulse;
∘ a pulse wave amplitude right by calculating a difference between amplitudes of the first peak and a second valley of the pulse;
∘ a pulse wave duration by calculating a difference between a time of the second valley and a time of the first valley of the pulse;
∘ a rise time by calculating a difference between a time of the first peak and a time of the first valley of the pulse;
∘ a systolic-to-diastolic duration ratio by calculating the ratio between a difference between the rise time and a difference between the time of the second valley and the time of the first peak of the pulse;
- eliminating a photoplethysmography pulse when:
∘ the rise time of said pulse is outside a predefined first range; or
∘ the pulse wave duration of said pulse is outside a predefined second range; or
∘ a ratio or an inverse ratio between the pulse wave amplitude right and the pulse wave amplitude left is smaller than a second threshold value; or
∘ the systolic-to-diastolic duration ratio is larger than a predefined third threshold value;
- calculating a signal quality index of the photoplethysmography segment by calculating a ratio of non-eliminated photoplethysmography pulses over a total number of photoplethysmography pulses comprised in said photoplethysmography segment.

8. The wearable device according to any one of the preceding claims **characterized in that** the wearable device is configured for eliminating the photoplethysmography segment when a signal quality index of said photoplethysmography signal is lower than a fourth threshold value.

9. The wearable device according to claim 8, **characterized in that** the wearable device is configured for setting said fourth threshold value at 20%.

10. The wearable device according to any one of the preceding claims, **characterized in that** the wearable device is configured for:
- collecting demographic information of the wearer, such as age, sex, body weight and height; and
- adding said demographic information into the cardiovascular profile vector of the wearer.

11. The wearable device according to any one of the preceding claims, **characterized in that** the wearable device is configured for grouping profile vectors of a plurality of wearers into a plurality of clusters based on a similarity in at least one of the biologically vital signs and/or based on a similarity in the demographic information of the wearers by using a silhouette coefficient to determine an optimal number of clusters for establishing a K-Means clustering on said profile vectors.

12. The wearable device according claim 11, **characterized in that** the wearable device is configured for training a Random Forest model on each cluster of profile vectors by combining a plurality of decision trees and training each decision tree on a separate subset of data of said cluster.

13. The wearable device according to any one of claims 11-12, **characterized in that** the wearable device is configured for:
- assigning a new wearer to one of the plurality of clusters based on at least one of the biologically vital signs of said new wearer and/or based on the demographic information of the new wearer; and
- using the Random Forest model corresponding to said cluster as a starting point, and fine-tuning said starting point to create a cardiovascular profile for the new wearer using a regularization framework such as a Lasso regression, a Ridge regression or an Elastic Net.

14. The wearable device according to any one of the preceding claims, **characterized in that** the wearable device is configured for updating the cardiovascular profile of the wearer by performing the steps of the preceding claims on new sets of acquired photoplethysmography segments.

15. The wearable device according to any one of the preceding claims, **characterized in that** said wearable device is configured for emitting light onto a skin of a wearer and receiving light emitted from the skin of the wearer wherein the device comprises a computer system configured to perform the operations according to any one of the preceding claims.

## Patentansprüche

1. Tragbare Vorrichtung zum Verarbeiten eines Photoplethysmographie-Signals, wobei die tragbare Vorrichtung dazu ausgebildet ist, Licht mit mehreren Lichtwellenlängen auszusenden, um einen Blutdruck eines Trägers der Vorrichtung zu messen, wobei das Photoplethysmographie-Signal eine Vielzahl von Pulsen aufweist und wobei ein Puls das Photoplethysmographie-Signal zwischen zwei aufeinanderfolgenden Tälern ist, **dadurch gekennzeichnet, dass** die tragbare Vorrichtung für zumindest eine Lichtwellenlänge dazu ausgebildet ist:
- ein Photoplethysmographie-Segment zu erstellen, indem sie das Photoplethysmographie-Signal innerhalb eines vordefinierten Zeitfensters erfasst;
- zumindest ein biologisches Vitalzeichen wie etwa eine Herzfrequenz des Trägers, eine Atemfrequenz des Trägers oder die Sauerstoffsättigungsrate des Trägers zu sammeln;
- zumindest einen wellenformbasierten Parameter, der proportional zu einer Spitzenamplitude des Photoplethysmographie-Segments ist, zu sammeln;
- zumindest einen zeitbasierten Parameter, der proportional zu einem Spitzenzeitpunkt des genannten Photoplethysmographie-Signals oder proportional zu einer Spitzenbreite des Photoplethysmographie-Segments ist, zu sammeln;
- einen kardiovaskulären Profilvektor des Trägers zu erstellen;
- einen mittleren quadratischen Fehler zwischen Komponenten des gesammelten Profilvektors und Komponenten eines Profilvektors, der zu einem zuvor vorhergesagten Cluster von Profilvektoren gehört, zu berechnen; und
- einen Blutdruck des Trägers unter Verwendung der in dem kardiovaskulären Profil des Trägers enthaltenen Photoplethysmographie-Segmente zu schätzen, wenn der mittlere quadratische Fehler kleiner als ein erster Schwellenwert ist.

2. Tragbare Vorrichtung gemäß Anspruch 1, **dadurch gekennzeichnet, dass** die tragbare Vorrichtung dazu ausgebildet ist, das Photoplethysmographie-Segment zu filtern, vorzugsweise unter Verwendung eines Bandpassfilters, und das gefilterte Photoplethysmographie-Segment in den verbleibenden Schritten des Verfahrens zu verwenden.

3. Tragbare Vorrichtung gemäß Anspruch 1 oder 2, **dadurch gekennzeichnet, dass** die tragbare Vorrichtung dazu ausgebildet ist, einen kardiovaskulären Profilvektor des Trägers zu erstellen, indem Photoplethysmographie-Segmente mit zumindest einem der folgenden Elemente verknüpft werden:
- dem gesammelten zumindest einen wellenformbasierten Parameter des Trägers;
- dem gesammelten zumindest einen zeitbasierten Parameter des Trägers; und
- dem gesammelten zumindest einen biologischen Vitalzeichen des Trägers.

4. Tragbare Vorrichtung gemäß einem der vorstehenden Ansprüche, **dadurch gekennzeichnet, dass** die tragbare Vorrichtung dazu ausgebildet ist, den gesammelten zumindest einen zeitbasierten Parameter durch Multiplizieren des Parameters mit einem Faktor, der umgekehrt proportional zu einer Spitzenbreite des Photoplethysmographie-Segments ist, zu normieren.

5. Tragbare Vorrichtung gemäß einem der vorstehenden Ansprüche, **dadurch gekennzeichnet, dass** die tragbare Vorrichtung dazu ausgebildet ist, Licht, das Lichtwellenlängen zwischen 400 nm und 1000 nm aufweist, zu emittieren.

6. Tragbare Vorrichtung gemäß einem der vorstehenden Ansprüche, **dadurch gekennzeichnet, dass** die tragbare Vorrichtung dazu ausgebildet ist, Licht, das
- eine Lichtwellenlänge von 525 nm;
- eine Lichtwellenlänge von 660 nm; und
- eine Lichtwellenlänge von 880 nm
aufweist, zu emittieren.

7. Tragbare Vorrichtung gemäß einem der vorstehenden Ansprüche, **dadurch gekennzeichnet, dass** die tragbare Vorrichtung für mindestens ein Photoplethysmographie-Segment dazu ausgebildet ist:
- für jeden Puls des Photoplethysmographie-Segments zumindest einen der folgenden Werte zu berechnen:
∘ eine Pulswellenamplitude links durch Berechnen einer Differenz zwischen Amplituden einer ersten Spitze und eines ersten Tals des Pulses;
∘ eine Pulswellenamplitude rechts durch Berechnen einer Differenz zwischen Amplituden der ersten Spitze und eines zweiten Tals des Pulses;
∘ eine Pulswellendauer durch Berechnen einer Differenz zwischen einer Zeit des zweiten Tals und einer Zeit des ersten Tals des Pulses;
∘ eine Anstiegszeit durch Berechnen einer Differenz zwischen einer Zeit der ersten Spitze und einer Zeit des ersten Tals des Pulses;
∘ ein Verhältnis der systolischen zur diastolischen Dauer durch Berechnen des Verhältnisses zwischen einer Differenz zwischen der Anstiegszeit und einer Differenz zwischen der Zeit des zweiten Tals und der Zeit der ersten Spitze des Pulses;
- einen Photoplethysmographie-Puls zu eliminieren, wenn:
∘ die Anstiegszeit des Pulses außerhalb eines vordefinierten ersten Bereichs liegt; oder
∘ die Pulswellendauer des Pulses außerhalb eines vordefinierten zweiten Bereichs liegt; oder
∘ ein Verhältnis oder ein umgekehrtes Verhältnis zwischen der Pulswellenamplitude rechts und der Pulswellenamplitude links kleiner als ein zweiter Schwellenwert ist; oder
∘ das Verhältnis der systolischen zur diastolischen Dauer größer als ein vordefinierter dritter Schwellenwert ist;
- einen Signalqualitätsindex des Photoplethysmographie-Segments durch Berechnen eines Verhältnisses nicht eliminierter Photoplethysmographie-Pulse zu einer Gesamtzahl von in dem Photoplethysmographie-Segment enthaltenen Photoplethysmographie-Pulsen zu berechnen.

8. Tragbare Vorrichtung gemäß einem der vorstehenden Ansprüche, **dadurch gekennzeichnet, dass** die tragbare Vorrichtung dazu ausgebildet ist, das Photoplethysmographie-Segment zu eliminieren, wenn ein Signalqualitätsindex des Photoplethysmographie-Signals niedriger als ein vierter Schwellenwert ist.

9. Tragbare Vorrichtung gemäß Anspruch 8, **dadurch gekennzeichnet, dass** die tragbare Vorrichtung dazu ausgebildet ist, den vierten Schwellenwert auf 20 % festzulegen.

10. Tragbare Vorrichtung gemäß einem der vorstehenden Ansprüche, **dadurch gekennzeichnet, dass** die tragbare Vorrichtung dazu ausgebildet ist:
- demografische Informationen des Trägers wie etwa Alter, Geschlecht, Körpergewicht und Körpergröße zu sammeln; und
- die demografischen Informationen dem kardiovaskulären Profilvektor des Trägers hinzuzufügen.

11. Tragbare Vorrichtung gemäß einem der vorstehenden Ansprüche, **dadurch gekennzeichnet, dass** die tragbare Vorrichtung dazu ausgebildet ist, Profilvektoren einer Vielzahl von Trägern basierend auf einer Ähnlichkeit bei zumindest einem der biologischen Vitalzeichen und/oder basierend auf einer Ähnlichkeit bei den demografischen Informationen der Träger in eine Vielzahl von Clustern zu gruppieren, indem ein Silhouettenkoeffizient verwendet wird, um eine optimale Anzahl von Clustern zum Bilden einer K-Means-Clusterung an den Profilvektoren zu bestimmen.

12. Tragbare Vorrichtung gemäß Anspruch 11, **dadurch gekennzeichnet, dass** die tragbare Vorrichtung zum Trainieren eines Random-Forest-Modells an jedem Cluster von Profilvektoren durch Kombinieren einer Vielzahl von Entscheidungsbäumen und Trainieren jedes Entscheidungsbaum an einer separaten Teilmenge von Daten des Clusters ausgebildet ist.

13. Tragbare Vorrichtung gemäß einem der Ansprüche 10-12, **dadurch gekennzeichnet, dass** die tragbare Vorrichtung dazu ausgebildet ist:
- einen neuen Träger basierend auf zumindest einem der biologischen Vitalzeichen des neuen Trägers und/oder basierend auf den demografischen Informationen über den neuen Träger einem der Vielzahl von Clustern zuzuordnen;
- und das diesem Cluster entsprechende Random-Forest-Modell als Ausgangspunkt zu verwenden und diesen Ausgangspunkt zu verfeinern, um ein kardiovaskuläres Profil für den neuen Träger unter Verwendung eines Regularisierungsrahmens wie etwa einer Lasso-Regression, einer Ridge-Regression oder eines Elastic Net zu erstellen.

14. Tragbare Vorrichtung gemäß einem der vorstehenden Ansprüche, **dadurch gekennzeichnet, dass** die tragbare Vorrichtung dazu ausgebildet ist, das kardiovaskuläre Profil des Trägers durch Durchführen der Schritte der vorstehenden Ansprüche an neuen Sätzen von erfassten Photoplethysmographie-Segmenten zu aktualisieren.

15. Tragbare Vorrichtung gemäß einem der vorstehenden Ansprüche, **dadurch gekennzeichnet, dass** die tragbare Vorrichtung dazu ausgebildet ist, Licht auf eine Haut eines Trägers zu emittieren und von der Haut des Trägers emittiertes Licht empfangen, wobei die Vorrichtung ein Computersystem, das dazu ausgebildet ist, die Operation gemäß einem der vorstehenden Ansprüche auszuführen, aufweist.

## Revendications

1. Dispositif portable destiné au traitement d'un signal de photopléthysmographie, ledit dispositif portable étant configuré pour émettre de la lumière à une pluralité de longueurs d'onde lumineuses afin de mesurer une pression artérielle d'un porteur dudit dispositif, le signal de photopléthysmographie comprenant une pluralité d'impulsions, une impulsion étant définie comme le signal de photopléthysmographie compris entre deux vallées consécutives, **caractérisé en ce que,** pour au moins une longueur d'onde lumineuse, le dispositif portable est configuré pour:
- créer un segment de photopléthysmographie en acquérant le signal de photopléthysmographie dans une fenêtre temporelle prédéfinie;
- collecter au moins un signe vital biologique, tel qu'une fréquence cardiaque du porteur, une fréquence respiratoire du porteur ou un taux de saturation en oxygène du porteur;
- collecter au moins un paramètre basé sur la forme d'onde proportionnel à une amplitude de pic dudit segment de photopléthysmographie;
- collecter au moins un paramètre temporel proportionnel à un instant de pic dudit signal de photopléthysmographie ou proportionnel à une largeur de pic dudit segment de photopléthysmographie;
- créer un vecteur de profil cardiovasculaire du porteur;
- calculer une erreur quadratique moyenne entre des composantes du vecteur de profil collecté et des composantes d'un vecteur de profil appartenant à un cluster de vecteurs de profils précédemment prédit; et
- estimer une pression artérielle du porteur à l'aide des segments de photopléthysmographie compris dans le profil cardiovasculaire du porteur lorsque ladite erreur quadratique moyenne est inférieure à une première valeur seuil.

2. Dispositif portable selon la revendication 1,
**caractérisé en ce que** le dispositif portable est configuré pour filtrer le segment de photopléthysmographie, de préférence au moyen d'un filtre passe-bande, et pour utiliser ledit segment de photopléthysmographie filtré dans les étapes restantes de ladite méthode.

3. Dispositif portable selon la revendication 1 ou 2, **caractérisé en ce que** le dispositif portable est configuré pour créer un vecteur de profil cardiovasculaire du porteur par concaténation de segments de photopléthysmographie avec au moins l'un des éléments suivants:
- ledit au moins un paramètre basé sur la forme d'onde collecté du porteur;
- ledit au moins un paramètre temporel collecté du porteur; et
- ledit au moins un signe vital biologique collecté du porteur.

4. Dispositif portable selon l'une quelconque des revendications précédentes,
**caractérisé en ce que** le dispositif portable est configuré pour normaliser ledit au moins un paramètre temporel collecté en multipliant ledit paramètre par un facteur inversement proportionnel à une largeur de pic du segment de photopléthysmographie.

5. Dispositif portable selon l'une quelconque des revendications précédentes,
**caractérisé en ce que** le dispositif portable est configuré pour émettre de la lumière comprenant des longueurs d'onde lumineuses comprises entre 400 nm et 1000 nm.

6. Dispositif portable selon l'une quelconque des revendications précédentes,
**caractérisé en ce que** le dispositif portable est configuré pour émettre de la lumière comprenant :
- une longueur d'onde lumineuse de 525 nm;
- une longueur d'onde lumineuse de 660 nm; et
- une longueur d'onde lumineuse de 880 nm.

7. Dispositif portable selon l'une quelconque des revendications précédentes,
**caractérisé en ce que,** pour au moins un segment de photopléthysmographie, le dispositif portable est configuré pour:
- calculer au moins l'une des valeurs suivantes pour chaque impulsion dudit segment de photopléthysmographie:
∘ une amplitude d'onde de pouls gauche en calculant une différence entre les amplitudes d'un premier pic et d'une première vallée de l'impulsion;
o∘ une amplitude d'onde de pouls droite en calculant une différence entre les amplitudes du premier pic et d'une seconde vallée de l'impulsion;
∘ une durée d'onde de pouls en calculant une différence entre un instant de la seconde vallée et un instant de la première vallée de l'impulsion;
∘ un temps de montée en calculant une différence entre un instant du premier pic et un instant de la première vallée de l'impulsion;
o un rapport de durée systole-diastole en calculant le rapport entre une différence correspondant au temps de montée et une différence entre l'instant de la seconde vallée et l'instant du premier pic de l'impulsion;
- éliminer une impulsion de photopléthysmographie lorsque:
∘ le temps de montée de ladite impulsion est en dehors d'une première plage prédéfinie; ou
∘ la durée d'onde de pouls de ladite impulsion est en dehors d'une seconde plage prédéfinie; ou
∘ un rapport ou un rapport inverse entre l'amplitude d'onde de pouls droite et l'amplitude d'onde de pouls gauche est inférieur à une seconde valeur seuil; ou
∘ le rapport de durée systole-diastole est supérieur à une troisième valeur seuil prédéfinie;
- calculer un indice de qualité du signal du segment de photopléthysmographie en calculant un rapport entre le nombre d'impulsions de photopléthysmographie non éliminées et le nombre total d'impulsions de photopléthysmographie comprises dans ledit segment de photopléthysmographie.

8. Dispositif portable selon l'une quelconque des revendications précédentes,
**caractérisé en ce que** le dispositif portable est configuré pour éliminer le segment de photopléthysmographie lorsqu'un indice de qualité du signal dudit signal de photopléthysmographie est inférieur à une quatrième valeur seuil.

9. Dispositif portable selon la revendication 8,
**caractérisé en ce que** le dispositif portable est configuré pour fixer ladite quatrième valeur seuil à 20 %.

10. Dispositif portable selon l'une quelconque des revendications précédentes,
**caractérisé en ce que** le dispositif portable est configuré pour:
- collecter des informations démographiques du porteur, telles que l'âge, le sexe, le poids corporel et la taille; et
- ajouter lesdites informations démographiques au vecteur de profil cardiovasculaire du porteur.

11. Dispositif portable selon l'une quelconque des revendications précédentes, **caractérisé en ce que** le dispositif portable est configuré pour regrouper des vecteurs de profils d'une pluralité de porteurs en une pluralité de clusters sur la base d'une similarité d'au moins l'un des signes vitaux biologiques et/ou sur la base d'une similarité des informations démographiques des porteurs, en utilisant un coefficient de silhouette afin de déterminer un nombre optimal de clusters pour établir un clustering de type K-means sur lesdits vecteurs de profils.

12. Dispositif portable selon la revendication 11, **caractérisé en ce que** le dispositif portable est configuré pour entraîner un modèle de forêt aléatoire sur chaque cluster de vecteurs de profils en combinant une pluralité d'arbres de décision et en entraînant chaque arbre de décision sur un sous-ensemble distinct de données dudit cluster.

13. Dispositif portable selon l'une quelconque des revendications 11-12,
**caractérisé en ce que** le dispositif portable est configuré pour:
- affecter un nouveau porteur à l'un de la pluralité de clusters sur la base d'au moins l'un des signes vitaux biologiques dudit nouveau porteur et/ou sur la base des informations démographiques dudit nouveau porteur; et
- utiliser le modèle de forêt aléatoire correspondant audit cluster comme point de départ, et affiner ledit point de départ afin de créer un profil cardiovasculaire pour le nouveau porteur au moyen d'un cadre de régularisation tel qu'une régression Lasso, une régression Ridge ou un Elastic Net.

14. Dispositif portable selon l'une quelconque des revendications précédentes,
**caractérisé en ce que** le dispositif portable est configuré pour mettre à jour le profil cardiovasculaire du porteur en exécutant les étapes des revendications précédentes sur de nouveaux ensembles de segments de photopléthysmographie acquis.

15. Dispositif portable selon l'une quelconque des revendications précédentes, **caractérisé en ce que** ledit dispositif portable est configuré pour émettre de la lumière sur une peau d'un porteur et pour recevoir de la lumière émise par la peau du porteur, le dispositif comprenant un système informatique configuré pour exécuter les opérations selon l'une quelconque des revendications précédentes.
